(19) Europäisches Patentamt / European Patent Office / Office européen des brevets

(11) **EP 2 812 075 B1**

(12) **EUROPÄISCHE PATENTSCHRIFT**

(45) Veröffentlichungstag und Bekanntmachung des Hinweises auf die Patenterteilung:
**24.01.2018 Patentblatt 2018/04**

(21) Anmeldenummer: **12781314.5**

(22) Anmeldetag: **05.11.2012**

(51) Int Cl.:
***A61Q 5/10*** *(2006.01)* ***A61K 8/73*** *(2006.01)*
***A61K 8/22*** *(2006.01)* ***A61K 8/81*** *(2006.01)*
***A45D 7/04*** *(2006.01)*

(86) Internationale Anmeldenummer:
**PCT/EP2012/071818**

(87) Internationale Veröffentlichungsnummer:
**WO 2013/087295 (20.06.2013 Gazette 2013/25)**

(54) **SCHONENDE OXIDATIVE HAARBEHANDLUNG MIT OXIDATIONSMITTEL UND SPEZIELLEM STÄRKEDERIVAT**

GENTLE OXIDATIVE HAIR TREATMENT WITH OXIDIZING AGENT AND SPECIAL STARCH DERIVATIVE

TRAITEMENT CAPILLAIRE RESPECTANT LES CHEVEUX À L'AIDE D'UN AGENT OXYDANT ET UN DÉRIVÉ D'AMIDON SPÉCIAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.12.2011 DE 102011088398**

(43) Veröffentlichungstag der Anmeldung:
**17.12.2014 Patentblatt 2014/51**

(73) Patentinhaber: **Henkel AG & Co. KGaA**
**40589 Düsseldorf (DE)**

(72) Erfinder: **SCHWEINSBERG, Matthias**
**22769 Hamburg (DE)**

(56) Entgegenhaltungen:
**EP-A2- 0 948 960** **EP-A2- 1 669 104**
**WO-A1-2005/082321** **WO-A1-2012/084904**
**DE-A1-102004 002 349** **JP-A- H0 517 322**
**US-A- 5 871 756** **US-A1- 2005 193 501**
**US-A1- 2006 075 580**

- **DATABASE WPI Week 201067 Thomson Scientific, London, GB; AN 2010-M48909 XP2728050, & JP 2010 215599 A (MANDOM KK) 30. September 2010 (2010-09-30)**
- **SINGH ET AL: "Factors influencing the physico-chemical, morphological, thermal and rheological properties of some chemically modified starches for food applications-A review", FOOD HYDROCOLLOIDS, ELSEVIER BV, NL, Bd. 21, Nr. 1, 1. Januar 2007 (2007-01-01) , Seiten 1-22, XP005664085, ISSN: 0268-005X, DOI: 10.1016/J.FOODHYD.2006.02.006**
- **SAOWAKON WATTANACHANT ET AL: "Effect of crosslinking reagents and hydroxypropylation levels on dual-modified sago starch properties", FOOD CHEMISTRY, Bd. 80, Nr. 4, 1. April 2003 (2003-04-01), Seiten 463-471, XP55132395, ISSN: 0308-8146, DOI: 10.1016/S0308-8146(02)00314-X**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 812 075 B1

## Beschreibung

Die vorliegende Erfindung betrifft Verfahren zur oxidativen Haarbehandlung sowie die darin verwendbaren Mittel, welche neben einem bestimmten Oxidationsmittel und einer bestimmten farbverändernden Komponente mindestens eine unvernetzte, mit Propylenoxid modifizierte Stärke enthalten.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten.

Die permanente, oxidative Färbung sowie die Haarbleiche und die Fixierung im Rahmen der Dauerwelle finden meist als oxidative Haarbehandlung in Gegenwart von Oxidationsmitteln, wie Wasserstoffperoxyd, statt. Das Oxidationsmittel erzielt hierbei leider nicht nur die erwünschte kosmetische Wirkung, sondern schädigt zusätzlich die Struktur des Haarkeratins. Das keratineigene Cystin oxidiert dabei zur Cysteinsäure, was sich negativ auf die Stabilität, die Haptik und die Optik der Haarfaser auswirkt. Derart geschädigtes Haar wirkt stumpf und spröde. In extremen Fällen kommt es sogar zum Haarbruch.

Aus der EP-A1-1 568 351 ist die Verwendung einer Zusammensetzung umfassend eine vorgelatinierte Amylose enthaltende Stärke zur Haltbarmachung künstlicher Farbe auf Haaren bekannt.

Die WO-A1-98/01109 betrifft Mittel zur Reinigung oder Pflege von Haar, enthaltend eine vorverkleisterte, vernetzte Stärke, ausgewählt aus einer ($C_2$ bis $C_6$)-Hydroxyalkylstärke und einer ($C_2$ bis $C_6$)-Acylstärke.

Weiterhin betreffen die WO 2012/084904 A1, die US 2005/0193501 A1 sowie die US 2006/0075580 A1 kosmetische Mittel zur oxidativen Haarfärbung, welche neben einem Oxidationsmittel sowie Oxidationsfarbstoffvorprodukten eine vernetzte, mittels Propylenoxid modifizierte Stärke enthalten.

Es ist die Aufgabe der vorliegenden Erfindung, die zuvor beschriebenen Nebenwirkungen oxidativer Haarbehandlungen bereits während der oxidativen Haarbehandlung zu verringern, ohne den Wirkungsgrad des oxidativen Kosmetikums, insbesondere bezüglich Farbintensität, Farbechtheit, Aufhellleistung bzw. Wellwirkung, zu verschlechtern.

Es wurde nun gefunden, daß die Aufgabe in hervorragendem Maße durch Mittel, enthaltend neben einem speziellen Oxidationsmittel sowie mindestens einem Oxidationsfarbstoffvorprodukt mindestens eine mittels Propylenoxid modifizierte unvernetzte Stärke, welche ein mittleres Molekulargewicht (Gewichtsmittel) von 50 bis 2500 kDa und einen Propylenoxidgehalt von 0,1 bis 20,0 Gew.-% (bezogen auf das Gewicht der mittels Propylenoxid modifizierten Stärke) aufweist, gelöst wird. Die Wirkung entfaltet sich während der oxidativen Haarbehandlung.

Ein erster Gegenstand der vorliegenden Erfindung sind daher Mittel, enthaltend in einem kosmetischen Träger eine Kombination aus

(i) mindestens einem Oxidationsmittel, wobei das Oxidationsmittel Wasserstoffperoxyd und/oder mindestens ein Anlagerungsprodukt von Wasserstoffperoxyd an anorganische oder organische Verbindungen ist und

(ii) mindestens einer mittels Propylenoxid modifizierten Stärke, welche ein mittleres Molekulargewicht (Gewichtsmittel) von 50 bis 2500 kDa und einen Propylenoxidgehalt von 0,1 bis 20,0 Gew.-% (bezogen auf das Gewicht der mittels Propylenoxid modifizierten Stärke) besitzt, wobei die mittels Propylenoxid modifizierte Stärke unvernetzt ist,

wobei es als farbverändernde Komponente mindestens ein Oxidationsfarbstoffvorprodukt vom Typ der Entwicklerkomponenten und gegebenenfalls zusätzlich mindestens eine Kupplerkomponente enthält.

Die Oxidationsmittel im Sinne der Erfindung sind von Luftsauerstoff verschieden und besitzen ein solches Oxidationspotenzial, dass es ermöglicht, Disulfidbrücken innerhalb oder zwischen den Proteinen des Haarkeratins zu knüpfen, und/oder das natürliche Farbpigment Melanin oxidativ aufzuhellen und/oder ein Oxidationsfarbstoffvorprodukt vom Entwicklertyp zu oxidieren.

Als Oxidationsmittel kommt erfindungsgemäß Wasserstoffperoxyd und/oder mindestens ein Anlagerungsprodukt davon, insbesondere an anorganische oder organische Verbindungen, wie beispielsweise Natriumperborat, Natriumpercarbonat, Magnesiumpercarbonat, Natriumpercarbamid, Polyvinylpyrrolidon $H_2O_2$ (n ist eine positive ganze Zahl größer 0), Harnstoffperoxid und Melaminperoxid in Frage.

Erfindungsgemäß kann das oxidative kosmetische Mittel auch zusammen mit einem Katalysator auf das Haar aufgebracht werden, der die Oxidation des Substrats, wie beispielsweise Oxidationsfarbstoffvorprodukte oder Melanin, aktiviert. Solche Katalysatoren sind z.B. Metallionen, Iodide, Chinone oder bestimmte Enzyme.

Geeignete Metallionen sind beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate.

Durch Verwendung dieser Metallsalze kann sowohl die Ausbildung einer Färbung beschleunigt als auch die Farbnuance gezielt beeinflusst werden.

Geeignete Enzyme sind z.B. Peroxidasen, die die Wirkung geringer Mengen an Wasserstoffperoxyd deutlich

verstärken können. Weiterhin sind solche Enzyme erfindungsgemäß geeignet, die mit Hilfe von Luftsauerstoff *in situ* geringe Mengen Wasserstoffperoxyd erzeugen und auf diese Weise die Oxidation der Farbstoffvorprodukte biokatalytisch aktivieren. Besonders geeignete Katalysatoren für die Oxidation von Farbstoffvorprodukten sind die sogenannten 2-Elektronen-Oxidoreduktasen in Kombination mit den dafür spezifischen Substraten, z.B.

- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das Oxidationsmittel ist bevorzugt in einer Menge von 1,0 bis 10 Gew.-%, insbesondere von 3,0 bis 10,0 Gew.-%, jeweils bezogen auf das Gewicht des anwendungsbereiten Mittels, in dem oxidativen kosmetischen Mittel enthalten.

Die besagte, mindestens eine mittels Propylenoxid modifizierte unvernetzte Stärke, wird bevorzugt während der oxidativen Haarbehandlung in Form eines Mittels, enthaltend in einem kosmetischen Träger mindestens eine mittels Propylenoxid modifizierte unvernetzte Stärke, welche ein mittleres Molekulargewicht (Gewichtsmittel) von 50 bis 2500 kDa und einen Propylenoxidgehalt von 0,1 bis 20,0 Gew.-% (bezogen auf das Gewicht der mittels Propylenoxid modifizierten Stärke) besitzt, verwendet.

Stärke ist ein Reservekohlenhydrat, das von vielen Pflanzen in Form von üblicherweise 1 bis 200 μm großen Stärkekörnern (Granula) in verschiedenen Pflanzenteilen gespeichert wird, z.B. in Knollen oder Wurzeln, Getreidesamen, Früchten sowie im Mark. Eine erfindungsgemäß verwendbare mittels Propylenoxid modifizierte Stärke wird sich vorzugsweise aus mindestens einer Stärke von Kartoffeln, Mais, Reis, Erbsen, Eicheln, Kastanien, Gerste, Weizen, Bananen, Sago, Hirse, Sorghum, Hafer, Gerste, Roggen, Bohnen, Batate, Maranta oder Maniok ableiten. Besonders ausgeprägte erfindungsgemäße Effekte werden durch entsprechende mittels Propylenoxid modifizierte Tapioka Stärke, mittels Propylenoxid modifizierte Kartoffelstärke, mittels Propylenoxid modifizierte Maisstärke oder Gemischen davon, erzielt. Es ist erfindungsgemäß ganz besonders bevorzugt als entsprechende mittels Propylenoxid modifizierte Stärke mittels Propylenoxid modifizierte Tapiokastärke einzusetzen.

Stärke gehört zu der Familie der Homoglycane und ist ein Polykondensationsprodukt von D-Glucose. Dabei besteht Stärke aus drei strukturell verschiedenen Polymeren der d-Glucopyranose, nämlich der Amylose, dem Amylopektin und einer sogenannten Zwischenfraktion. Höhere Pflanzen enthalten zwischen 0 bis 45 Gew.-% Amylose bezogen auf die Trockensubstanz.

Die Zwischenfraktion, die auch als anormales Amylopektin bezeichnet wird, steht strukturell zwischen der Amylose und dem Amylopektin. Die im Rahmen dieser Anmeldung definierten Mengenangaben für Amylopektin umfassen die Zwischenfraktion.

Es ist erfindungsgemäß bevorzugt, wenn die mittels Propylenoxid modifizierte Stärke einen Amylosegehalt von kleiner 25 Gew.-%, insbesondere von kleiner 20 Gew.-%, - jeweils bezogen auf das Gewischt der besagten Stärke - besitzt. Es zeigte sich, dass sich zur Erreichung des erfindungsgemäßen Effektes besonders eine Stärke eignet, die 17 bis 22 Gew.-% Amylose und 78 bis 83 Gew.-% Amylopektin enthält.

Amylose besteht aus überwiegend linear α-1,4-glycosidisch verknüpfter d-Glucose, $M_r$ 50000-150000. Die resultierenden Ketten bilden in der Stärke Doppelhelices.

Amylopektin enthält neben den für Amylose beschriebenen α-1,4-Verknüpfungen auch in einer Menge von 4 bis 6% α-1,6-Bindungen als Verzweigungsstellen. Der durchschnittliche Abstand zwischen den Verzweigungsstellen beträgt etwa 12 bis 17 Glucose-Einheiten. Die Molmasse von $10^7$ bis $7 \cdot 10^8$ entspricht ca. $10^5$ Glucose-Einheiten, womit Amylopektin zu den größten Biopolymeren gehört. Besagte Verzweigungen sind derart über das Molekül verteilt, daß sich eine

Büschelstruktur mit relativ kurzen Seitenketten entwickelt. Zwei dieser Seitenketten bilden jeweils eine Doppelhelix. Durch die vielen Verzweigungsstellen ist Amylopektin in Wasser relativ gut löslich.

Unter einer mittels Propylenoxid modifizierten Stärke wird erfindungsgemäß ein Reaktionsprodukt einer Stärke mit Propylenoxid verstanden. Ein solches Reaktionsprodukt umfasst mindestens eine Struktureinheit der Formel (I),

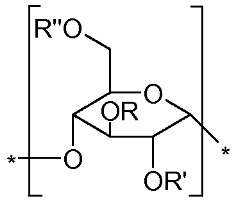

(I),

worin mindestens ein Rest R, R' oder R" für eine Gruppe der Formel

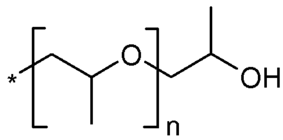

mit n ≥0 steht

und maximal 2 der Reste aus R, R', R" für ein Wasserstoffatom steht. Eine mit dem Symbol * gekennzeichnete Bindung in Formeln dieser Anmeldung entspricht einer freien Valenz der entsprechenden Struktureinheit. Die entsprechend mittels Propylenoxid modifizierten Stärken werden beispielsweise durch Umsetzung einer nativen Stärke mit Propylenoxid bereitgestellt. Vor der Modifikation mittels Propylenoxid kann die Stärke diversen physikalischen oder chemischen Prozessen ausgesetzt worden sein, wie z.B. einer Wärmebehandlung, Scherung, einer thermischen, säurehydrolytischen, oxidativen oder enzymatischen Spaltung etc.

Es ist erfindungsgemäß bevorzugt, wenn die mittels Propylenoxid modifizierte Stärke in dem erfindungsgemäßen Mittel nicht in Form einzelner Stärkekörnchen (Granula) vorliegt. Zu diesem Zweck werden die Stärkekörner aufgeschlossen z.B. durch Hitze oder Scherung und die entsprechenden Polysaccharidmoleküle aus dem Verbund freigesetzt. Die freigesetzten Polysaccharidmoleküle werden nach oder vor der Freisetzung mit Propylenoxid modifiziert.

Im Rahmen einer bevorzugten Ausführungsform ist die mittels Propylenoxid modifizierte Stärke verkleistert. Wird eine wässrige Suspension von Stärke erhitzt oder komprimiert, so beobachtet man bei einer kritischen Temperatur bzw. Druck eine tangentiale Quellung der Körper mit Verlust der Doppelbrechung, Änderung der Röntgenstruktur und abrupter Steigerung der Viskosität der Lösung. Diese Erscheinung wird Verkleisterung genannt.

Die erfindungsgemäßen mittels Propylenoxid modifizierten Stärken liegen in dem erfindungsgemäßen Mittel in einer Molekulargewichtsverteilung vor. Die Molekulargewichtsverteilung wurde experimentell mittels Gelfiltrationschromatographie gegen Dextran bestimmt. Ein wichtiges Merkmal der Erfindung ist das Gewichtsmittel des mittleren Molekulargewichts der in dem erfindungsgemäßen Mittel enthaltenen mittels Propylenoxid modifizierten Stärken. Das besagte Gewichtsmittel ist ein mittleres Molekulargewicht, welches das Totalgewicht der Moleküle verschiedenen Molekulargewichts berücksichtigt und nicht lediglich nur die Anzahl der Moleküle. Zur statistischen Berechnung des Gewichtsmittels wird zunächst der "Gewichtsbruch"

$$w_i = (N_i M_i) / [\Sigma(N_i M_i)]$$

definiert. Dieser gibt den Gewichtsanteil an Makromolekülen in der Probe an, die aus i Segmenten (z. B. Monomerbausteinen) der Masse $M_i$ bestehen und in der Probe $N_i$-mal vorkommen. Für das Gewichtsmittel des Molekulargewichts $M_w = \sum w_i M_i$ gilt damit

$$M_w = [\Sigma(N_i M^2_i)] / [\Sigma(N_i M_i)].$$

Besonders Bevorzugte erfindungsgemäße Mittel enthalten solche besagten mittels Propylenoxid modifizierten Stärken, die ein mittleres Molekulargewicht (Gewichtsmittel) von 100 bis 2000 kDa, insbesondere von 500 bis 1800 kDa, ganz

besonders bevorzugt von 700 bis 1000 kDa, aufweisen. Erfindungsgemäß ist die mittels Propylenoxid modifizierte Stärke unvernetzt. Eine Vernetzung der mittels Propylenoxid modifizierten Stärke liegt vor, wenn die linearen bzw. verzweigten Polysaccharid-Makromoleküle der Stärke kovalent durch ein Vernetzungsmittel unter Bildung eines dreidimensionalen, unlöslichen und nur noch quellbaren polymeren Netzwerkes verknüpft werden. Native Stärke gilt im Allgemeinen als unvernetzt und bedarf - falls eine Vernetzung gewünscht wäre - einer künstlichen Vernetzung mittels Synthesechemie. Eine solche künstliche Vernetzung lässt sich mit Vernetzungsmitteln wie beispielsweise mit Epichlorhydrin durchführen. (Mittels Propylenoxid modifizierte) Stärken, die eine solche Vernetzung nicht aufweisen, sind unvernetzt. Zur Erzielung eines niedrigeren Molekulargewichts z.B. von 700-900 kDa, werden besagte Stärken einer mechanischen Spaltung, einer enzymatischen Spaltung (insbesondere mit alpha-Amylase, beta-Amylase, Glucoamylase oder entzweigende Enzyme), einer säurehydrolytischen Spaltung (insbesondere mit Salzsäure, Schwefelsäure oder Phosphorsäure), einer thermischen Spaltung oder einer Umsetzung mit Oxidationsmitteln (wie Periodat, Hypochlorit, Chromsäure, Permanganat, Stickstoffdioxid, Wasserstoffperoxid oder organischer Percarbonsäure, bevorzugt mit Wasserstoffperoxid), ausgesetzt. Zur mechanischen Spaltung der Stärke eignen sich Kneter, Extruder, Stator/Rotor-Maschinen und/oder Rührwerke. Die oxidative Spaltung mittels Wasserstoffperoxid eignet sich bevorzugt. Zu diesem Zweck wird beispielsweise die mittels Propylenoxid modifizierte Stärke in Wasser gegeben, auf 50 bis 70 °C erhitzt, Wasserstoffperoxid zugefügt und bei 70 bis 85°C für 2 bis 5 Stunden gerührt.
Der Gehalt an Propylenoxid der Stärke wirkt sich auf die Feinabstimmung der Eigenschaften des erhaltenen Haarschutzes und der Stabilität der kosmetischen Mittel aus. Es zeigte sich, dass die Parameter weiter optimiert werden, wenn die besagte mittels Propylenoxid modifizierte Stärke - bezogen auf das Gewicht der modifizierten Stärke - einen Propylenoxidgehalt von 2 bis 12 Gew.-%, besonders bevorzugt einen Propylenoxidgehalt von 3,0 bis 10,0 Gew.-%, ganz besonders bevorzugt einen Propylenoxidgehalt von 4,0 bis 6,0 Gew.-%, aufweist. Der Propylenoxidgehalt lässt sich beispielsweise nach Durchführung einer Hodges-Spaltung mit der Methode gemäß DIN EN 13268 bestimmen.
Ferner hat es sich erwiesen, dass solche kosmetischen Mittel sich im Sinne der Erfindung hervorragend eignen, in denen die besagte mittels Propylenoxid modifizierte Stärke in einer 43 Gew.-%-igen Lösung in Wasser (i.e. einer 43 Gew.-%-igen wässrigen Lösung) eine Viskosität im Bereich von 150 bis 1500000 mPa·s (Brookfield Viskosimeter, Spindel #7 bei 20°C und 20 U/min) aufweist. Hervorragend geeignete Propylenoxid modifizierte Polysaccharide weisen Viskositäten von 3000 bis 200000 mPa·s, insbesondere von 10000 bis 100000 mPa·s, ganz besonders bevorzugt von 40000 bis 70000 mPa·s, (jeweils gemessen unter den zuvor genannten Bedingungen) auf.
Die besagte mittels Propylenoxid modifizierte Stärke wird bevorzugt in einem kosmetischen Träger verwendet. Als kosmetische Träger eignen sich erfindungsgemäß besonders Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind. Es ist aber auch denkbar, die Inhaltsstoffe in eine pulverförmige oder auch tablettenförmige Formulierung zu integrieren, welche vor der Anwendung in Wasser gelöst wird. Die kosmetischen Träger können insbesondere wässrig oder wässrig-alkoholisch sein.

Ein wässriger kosmetischer Träger enthält mindestens 50 Gew.-% Wasser.

Unter wässrig-alkoholischen kosmetischen Trägern sind im Sinne der vorliegenden Erfindung wässrige Lösungen enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Erfindungsgemäß ganz besonders bevorzugte kosmetische Mittel gehorchen mindestens einer der folgenden Ausführungsformen A) bis R):

A): Kosmetisches Mittel zur Färbung keratinischer Fasern, insbesondere menschlicher Haare, enthaltend in einem kosmetischen Träger mindestens ein Oxidationsmittel und mindestens eine unvernetzte, mittels Propylenoxid modifizierte Stärke, welche ein mittleres Molekulargewicht (Gewichtsmittel) von 50 bis 2500 kDa und einen Propylenoxidgehalt von 2,0 bis 12,0 Gew.-% (bezogen auf das Gewicht der mittels Propylenoxid modifizierten Stärke) aufweist.

B): Kosmetisches Mittel zur Färbung keratinischer Fasern, insbesondere menschlicher Haare, enthaltend in einem kosmetischen Träger mindestens ein Oxidationsmittel und mindestens eine unvernetzte, mittels Propylenoxid modifizierte Stärke, welche ein mittleres Molekulargewicht (Gewichtsmittel) von 700 bis 1000 kDa und einen Propylenoxidgehalt von 2,0 bis 12,0 Gew.-% (bezogen auf das Gewicht der mittels Propylenoxid modifizierten Stärke) aufweist.

C): Kosmetisches Mittel zur Färbung keratinischer Fasern, insbesondere menschlicher Haare, enthaltend in einem kosmetischen Träger mindestens ein Oxidationsmittel und mindestens eine unvernetzte, mittels Propylenoxid modifizierte Stärke, welche ein mittleres Molekulargewicht (Gewichtsmittel) von 50 bis 2500 kDa, einen Propylenoxidgehalt von 2,0 bis 12,0 Gew.-% (bezogen auf das Gewicht der mittels Propylenoxid modifizierten Stärke) und eine Viskosität von 3000 bis 200000 mPas (in 43 Gew.-%-iger wässriger Lösung, Brookfield Viskosimeter, Spindel #7

bei 20°C und 20 U/min) aufweist.

D): Kosmetisches Mittel zur Färbung keratinischer Fasern, insbesondere menschlicher Haare, enthaltend in einem kosmetischen Träger mindestens ein Oxidationsmittel und mindestens eine unvernetzte, mittels Propylenoxid modifizierte Stärke, welche ein mittleres Molekulargewicht (Gewichtsmittel) von 50 bis 2500 kDa, einen Propylenoxidgehalt von 2,0 bis 12,0 Gew.-% (bezogen auf das Gewicht der mittels Propylenoxid modifizierten Stärke) und eine Viskosität von 1000 bis 100000 mPas (in 43 Gew.-%-iger wässriger Lösung, Brookfield Viskosimeter, Spindel #7 bei 20°C und 20 U/min) aufweist.

E): Kosmetisches Mittel zur Färbung keratinischer Fasern, insbesondere menschlicher Haare, enthaltend in einem kosmetischen Träger mindestens ein Oxidationsmittel und mindestens eine unvernetzte, mittels Propylenoxid modifizierte Stärke, welche ein mittleres Molekulargewicht (Gewichtsmittel) von 700 bis 1000 kDa, einen Propylenoxidgehalt von 2,0 bis 12,0 Gew.-% (bezogen auf das Gewicht der mittels Propylenoxid modifizierten Stärke) und eine Viskosität von 3000 bis 200000 mPas (in 43 Gew.-%-iger wässriger Lösung, Brookfield Viskosimeter, Spindel #7 bei 20°C und 20 U/min) aufweist.

F): Kosmetisches Mittel zur Färbung keratinischer Fasern, insbesondere menschlicher Haare, enthaltend in einem kosmetischen Träger mindestens ein Oxidationsmittel und mindestens eine unvernetzte, mittels Propylenoxid modifizierte Stärke, welche ein mittleres Molekulargewicht (Gewichtsmittel) von 700 bis 1000 kDa, einen Propylenoxidgehalt von 2,0 bis 12,0 Gew.-% (bezogen auf das Gewicht der mittels Propylenoxid modifizierten Stärke) und eine Viskosität von 10000 bis 100000 mPas (in 43 Gew.-%-iger wässriger Lösung, Brookfield Viskosimeter, Spindel #7 bei 20°C und 20 U/min) aufweist.

G): Kosmetisches Mittel zur Färbung keratinischer Fasern, insbesondere menschlicher Haare, enthaltend in einem kosmetischen Träger ein Oxidationsmittel und mindestens eine unvernetzte, mittels Propylenoxid modifizierte Stärke ausgewählt aus Tapiokastärke, Kartoffelstärke, Maisstärke oder Mischungen, wobei die besagte modifizierte Stärke ein mittleres Molekulargewicht (Gewichtsmittel) von 50 bis 2500 kDa und einen Propylenoxidgehalt von 2,0 bis 12,0 Gew.-% (bezogen auf das Gewicht der mittels Propylenoxid modifizierten Stärke) aufweist.

H): Kosmetisches Mittel zur Färbung keratinischer Fasern, insbesondere menschlicher Haare, enthaltend in einem kosmetischen Träger mindestens ein Oxidationsmittel und mindestens eine unvernetzte, mittels Propylenoxid modifizierte Stärke ausgewählt aus Tapiokastärke, Kartoffelstärke, Maisstärke oder Mischungen, wobei die besagte modifizierte Stärke ein mittleres Molekulargewicht (Gewichtsmittel) von 700 bis 1000 kDa und einen Propylenoxidgehalt von 2,0 bis 12,0 Gew.-% (bezogen auf das Gewicht der mittels Propylenoxid modifizierten Stärke) aufweist.

I): Kosmetisches Mittel zur Färbung keratinischer Fasern, insbesondere menschlicher Haare, enthaltend in einem kosmetischen Träger mindestens ein Oxidationsmittel und mindestens eine unvernetzte, mittels Propylenoxid modifizierte Stärke ausgewählt aus Tapiokastärke, Kartoffelstärke, Maisstärke oder Mischungen, wobei die besagte modifizierte Stärke ein mittleres Molekulargewicht (Gewichtsmittel) von 50 bis 2500 kDa, einen Propylenoxidgehalt von 2,0 bis 12,0 Gew.-% (bezogen auf das Gewicht der mittels Propylenoxid modifizierten Stärke) und eine Viskosität von 3000 bis 200000 mPas (in 43 Gew.-%-iger wässriger Lösung, Brookfield Viskosimeter, Spindel #7 bei 20°C und 20 U/min) aufweist.

J): Kosmetisches Mittel zur Färbung keratinischer Fasern, insbesondere menschlicher Haare, enthaltend in einem kosmetischen Träger mindestens ein Oxidationsmittel und mindestens eine unvernetzte, mittels Propylenoxid modifizierte Stärke ausgewählt aus Tapiokastärke, Kartoffelstärke, Maisstärke oder Mischungen, wobei die besagte modifizierte Stärke ein mittleres Molekulargewicht (Gewichtsmittel) von 50 bis 2500 kDa, einen Propylenoxidgehalt von 2,0 bis 12,0 Gew.-% (bezogen auf das Gewicht der mittels Propylenoxid modifizierten Stärke) und eine Viskosität von 1000 bis 100000 mPas (in 43 Gew.-%-iger wässriger Lösung, Brookfield Viskosimeter, Spindel #7 bei 20°C und 20 U/min) aufweist.

K): Kosmetisches Mittel zur Färbung keratinischer Fasern, insbesondere menschlicher Haare, enthaltend in einem kosmetischen Träger mindestens ein Oxidationsmittel und mindestens eine unvernetzte, mittels Propylenoxid modifizierte Stärke ausgewählt aus Tapiokastärke, Kartoffelstärke, Maisstärke oder Mischungen, wobei die besagte modifizierte Stärke ein mittleres Molekulargewicht (Gewichtsmittel) von 700 bis 1000 kDa, einen Propylenoxidgehalt von 2,0 bis 12,0 Gew.-% (bezogen auf das Gewicht der mittels Propylenoxid modifizierten Stärke) und eine Viskosität von 3000 bis 200000 mPas (in 43 Gew.-%-iger wässriger Lösung, Brookfield Viskosimeter, Spindel #7 bei 20°C und 20 U/min) aufweist.

L): Kosmetisches Mittel zur Färbung keratinischer Fasern, insbesondere menschlicher Haare, enthaltend in einem kosmetischen Träger mindestens ein Oxidationsmittel und mindestens eine unvernetzte, mittels Propylenoxid modifizierte Stärke ausgewählt aus Tapiokastärke, Kartoffelstärke, Maisstärke oder Mischungen, wobei die besagte modifizierte Stärke ein mittleres Molekulargewicht (Gewichtsmittel) von 700 bis 1000 kDa, einen Propylenoxidgehalt von 2,0 bis 12,0 Gew.-% (bezogen auf das Gewicht der mittels Propylenoxid modifizierten Stärke) und eine Viskosität von 10000 bis 100000 mPas (in 43 Gew.-%-iger wässriger Lösung, Brookfield Viskosimeter, Spindel #7 bei 20°C und 20 U/min) aufweist.

M): Kosmetisches Mittel zur Färbung keratinischer Fasern, insbesondere menschlicher Haare, enthaltend in einem

kosmetischen Träger mindestens ein Oxidationsmittel und mindestens eine unvernetzte, mittels Propylenoxid modifizierte Tapiokastärke, welche ein mittleres Molekulargewicht (Gewichtsmittel) von 50 bis 2500 kDa und einen Propylenoxidgehalt von 2,0 bis 12,0 Gew.-% (bezogen auf das Gewicht der mittels Propylenoxid modifizierten Tapiokastärke) aufweist.
N): Kosmetisches Mittel zur Färbung keratinischer Fasern, insbesondere menschlicher Haare, enthaltend in einem kosmetischen Träger mindestens ein Oxidationsmittel und mindestens eine unvernetzte, mittels Propylenoxid modifizierte Tapiokastärke, welche ein mittleres Molekulargewicht (Gewichtsmittel) von 700 bis 1000 kDa und einen Propylenoxidgehalt von 2,0 bis 12,0 Gew.-% (bezogen auf das Gewicht der mittels Propylenoxid modifizierten Tapiokastärke) aufweist.
O): Kosmetisches Mittel zur Färbung keratinischer Fasern, insbesondere menschlicher Haare, enthaltend in einem kosmetischen Träger mindestens ein Oxidationsmittel und mindestens eine unvernetzte, mittels Propylenoxid modifizierte Tapiokastärke, welche ein mittleres Molekulargewicht (Gewichtsmittel) von 50 bis 2500 kDa, einen Propylenoxidgehalt von 2,0 bis 12,0 Gew.-% (bezogen auf das Gewicht der mittels Propylenoxid modifizierten Tapiokastärke) und eine Viskosität von 3000 bis 200000 mPas (in 43 Gew.-%-iger wässriger Lösung, Brookfield Viskosimeter, Spindel #7 bei 20°C und 20 U/min) aufweist.
P): Kosmetisches Mittel zur Färbung keratinischer Fasern, insbesondere menschlicher Haare, enthaltend in einem kosmetischen Träger mindestens ein Oxidationsmittel und mindestens eine unvernetzte, mittels Propylenoxid modifizierte Tapiokastärke, welche ein mittleres Molekulargewicht (Gewichtsmittel) von 50 bis 2500 kDa, einen Propylenoxidgehalt von 2,0 bis 12,0 Gew.-% (bezogen auf das Gewicht der mittels Propylenoxid modifizierten Tapiokastärke) und eine Viskosität von 1000 bis 100000 mPas (in 43 Gew.-%-iger wässriger Lösung, Brookfield Viskosimeter, Spindel #7 bei 20°C und 20 U/min) aufweist.
Q): Kosmetisches Mittel zur Färbung keratinischer Fasern, insbesondere menschlicher Haare, enthaltend in einem kosmetischen Träger mindestens ein Oxidationsmittel und mindestens eine unvernetzte, mittels Propylenoxid modifizierte Tapiokastärke, welche ein mittleres Molekulargewicht (Gewichtsmittel) von 700 bis 1000 kDa, einen Propylenoxidgehalt von 2,0 bis 12,0 Gew.-% (bezogen auf das Gewicht der mittels Propylenoxid modifizierten Tapiokastärke) und eine Viskosität von 3000 bis 200000 mPas (in 43 Gew.-%-iger wässriger Lösung, Brookfield Viskosimeter, Spindel #7 bei 20°C und 20 U/min) aufweist.
R): Kosmetisches Mittel zur Färbung keratinischer Fasern, insbesondere menschlicher Haare, enthaltend in einem kosmetischen Träger mindestens ein Oxidationsmittel und mindestens eine unvernetzte, mittels Propylenoxid modifizierte Tapiokastärke, welche ein mittleres Molekulargewicht (Gewichtsmittel) von 700 bis 1000 kDa, einen Propylenoxidgehalt von 2,0 bis 12,0 Gew.-% (bezogen auf das Gewicht der mittels Propylenoxid modifizierten Tapiokastärke) und eine Viskosität von 10000 bis 100000 mPas (in 43 Gew.-%-iger wässriger Lösung, Brookfield Viskosimeter, Spindel #7 bei 20°C und 20 U/min) aufweist.

Die Ausführungsformen A) bis R) des erfindungsgemäßen kosmetischen Mittels enthalten als Oxidationsmittel Wasserstoffperoxid und/oder mindestens ein Anlagerungsprodukt davon an anorganische oder organische Verbindungen sowie als farbverändernde Komponente mindestens ein Oxidationsfarbstoffvorprodukt vom Typ der Entwicklerkomponenten und gegebenenfalls zusätzlich mindestens eine Kupperkomponente.

Die erfindungsgemäßen kosmetischen Mittel (bevorzugt die kosmetischen Mittel der Ausführungsformen A) bis R)) enthalten die besagten mittels Propylenoxid modifizierten Stärken bevorzugt in einer Menge von in einer Menge von 0,01 Gew.-% bis 20 Gew.-%, besonders bevorzugt von 0,01 Gew.-% bis 10,0 Gew.-%, ganz besonders bevorzugt von 1 Gew.-% bis 5 Gew.-%, am bevorzugtesten von 0,1 bis 2,0 Gew.-%, jeweils bezogen auf das Gewicht des anwendungsbereiten Mittels.
Erfindungsgemäß wird das erfindungsgemäße Mittel im Rahmen einer Farbveränderung der Haare verwendet. Zu diesem Zweck enthalten die kosmetischen Mittel daher mindestens eine farbverändernde Komponente.
Die farbverändernden Komponenten im Sinne der vorliegenden Erfindung werden bevorzugt ausgewählt

(1) aus mindestens einem Oxidationsfarbstoffvorprodukt vom Typ der Entwicklerkomponenten und gegebenenfalls zusätzlich mindestens einer Kupplerkomponente.
und/oder
(2) aus mindestens einem direktziehenden Farbstoff
und/oder
(3) aus mindestens einer Vorstufe naturanaloger Farbstoffe.

Erfindungsgemäße farbgebende Verbindungen sind ausgewählt aus mindestens einem Oxidationsfarbstoffvorprodukt vom Typ der Entwicklerkomponenten und gegebenenfalls zusätzlich mindestens einer Kupplerkomponente.
Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische

Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (Ent1)

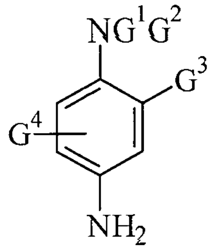

(Ent1)

wobei

- $G^1$ steht für ein Wasserstoffatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen 4'-Aminophenylrest oder einen $C_1$-bis $C_4$-Alkylrest, der mit einer stickstoffhaltigen Gruppe, einem Phenyl- oder einem 4'-Aminophenylrest substituiert ist;
- $G^2$ steht für ein Wasserstoffatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest oder einen $C_1$- bis $C_4$-Alkylrest, der mit einer stickstoffhaltigen Gruppe substituiert ist;
- $G^3$ steht für ein Wasserstoffatom, ein Halogenatom, wie ein Chlor-, Brom-, Iod- oder Fluoratom, einen $C_1$- bis $C_4$-Alkylrest, einen $C_1$- bis $C_4$-Monohydroxyalkylrest, einen $C_2$- bis $C_4$-Polyhydroxyalkylrest, einen $C_1$- bis $C_4$-Hydroxyalkoxyrest, einen $C_1$- bis $C_4$-Acetylaminoalkoxyrest, einen $C_1$- bis $C_4$- Mesylaminoalkoxyrest oder einen $C_1$- bis $C_4$-Carbamoylaminoalkoxyrest;
- $G^4$ steht für ein Wasserstoffatom, ein Halogenatom oder einen $C_1$- bis $C_4$-Alkylrest oder
- wenn $G^3$ und $G^4$ in ortho-Stellung zueinander stehen, können sie gemeinsam eine verbrückende $\alpha,\omega$-Alkylendioxogruppe, wie beispielsweise eine Ethylendioxygruppe bilden.

Beispiele für die als Substituenten in den erfindungsgemäßen Verbindungen genannten $C_1$- bis $C_4$-Alkylreste sind die Gruppen Methyl, Ethyl, Propyl, Isopropyl und Butyl. Ethyl und Methyl sind bevorzugte Alkylreste. Erfindungsgemäß bevorzugte $C_1$- bis $C_4$-Alkoxyreste sind beispielsweise eine Methoxy- oder eine Ethoxygruppe. Weiterhin können als bevorzugte Beispiele für eine $C_1$- bis $C_4$-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte $C_2$- bis $C_4$-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (Ent1) sind insbesondere die Aminogruppen, $C_1$- bis $C_4$-Monoalkylaminogruppen, $C_1$- bis $C_4$-Dialkylaminogruppen, $C_1$- bis $C_4$-Trialkylammoniumgruppen, $C_1$- bis $C_4$-Monohydroxyalkylaminogruppen, Imidazolinium und Ammonium.

Besonders bevorzugte p-Phenylendiamine der Formel (Ent1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (Ent1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Mitteln gemäß der Erfindung verwendet werden

können, kann man insbesondere die Verbindungen nennen, die der folgenden Formel (Ent2) entsprechen, sowie ihre physiologisch verträglichen Salze:

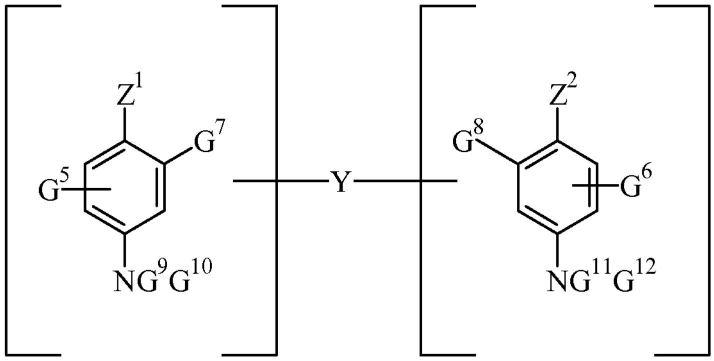

(Ent2)

wobei:

- $Z^1$ und $Z^2$ stehen unabhängig voneinander für einen Hydroxyl- oder $NH_2$-Rest, der gegebenenfalls durch einen $C_1$- bis $C_4$-Alkylrest, durch einen $C_1$- bis $C_4$-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder $C_1$- bis $C_8$-Alkoxyreste substituiert sein kann, oder eine direkte Bindung,
- $G^5$ und $G^6$ stehen unabhängig voneinander für ein Wasserstoff- oder Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen $C_1$- bis $C_4$-Aminoalkylrest oder eine direkte Verbindung zur Verbrückung Y,
- $G^7$, $G^8$, $G^9$, $G^{10}$, $G^{11}$ und $G^{12}$ stehen unabhängig voneinander für ein Wasserstoffatom, eine direkte Bindung zur Verbrückung Y oder einen $C_1$- bis $C_4$-Alkylrest,
  mit den Maßgaben, dass
- die Verbindungen der Formel (Ent2) nur eine Verbrückung Y pro Molekül enthalten und
- die Verbindungen der Formel (Ent2) mindestens eine Aminogruppe enthalten, die mindestens ein Wasserstoffatom trägt.

Die in Formel (Ent2) verwendeten Substituenten sind erfindungsgemäß analog zu den obigen Ausführungen definiert.

Bevorzugte zweikernige Entwicklerkomponenten der Formel (Ent2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (Ent2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (Ent3)

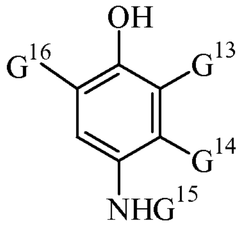

(Ent3)

wobei:

- $G^{13}$ steht für ein Wasserstoffatom, ein Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen $C_1$- bis $C_4$-Aminoalkylrest, einen Hydroxy-($C_1$- bis $C_4$)-alkylaminorest, einen $C_1$- bis $C_4$-Hydroxyalkoxyrest, einen $C_1$- bis $C_4$-Hydroxyalkyl-($C_1$-bis $C_4$)-aminoalkylrest oder einen (Di-$C_1$- bis $C_4$-Alkylamino)-($C_1$- bis $C_4$)-alkylrest, und
- $G^{14}$ steht für ein Wasserstoff- oder Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen $C_1$- bis $C_4$-Aminoalkylrest oder einen $C_1$- bis $C_4$-Cyanoalkylrest,
- $G^{15}$ steht für Wasserstoff, einen $C_1$- bis $C_4$-Alkylrest, einen $C_1$- bis $C_4$-Monohydroxyalkylrest, einen $C_2$- bis $C_4$-Polyhydroxyalkylrest, einen Phenylrest oder einen Benzylrest, und
- $G^{16}$ steht für Wasserstoff oder ein Halogenatom.

Die in Formel (Ent3) verwendeten Substituenten sind erfindungsgemäß analog zu den obigen Ausführungen definiert.

Bevorzugte p-Aminophenole der Formel (Ent3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (Ent3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-PyrimidinDerivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)amino-3-amino-pyridin, 2,3-Diamino-6-methoxy-pyridin, 2-(ß-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(ß-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(ß-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(ß-hydroxyethyl)amino-1-methylpyrazol.

Als Kupplerkomponenten werden in der Regel m-Phenylendiaminderivate, Naphthole, Resorcin und Resorcinderivate, Pyrazolone und m-Aminophenolderivate sowie heterozyklische Verbindungen verwendet.

Erfindungsgemäß bevorzugte Kupplerkomponenten sind

- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol

sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin und die physiologisch verträglichen Salzen der vorgenannten Verbindungen.

Die erfindungsgemäßen, kosmetischen Mittel enthalten die Entwicklerkomponenten bevorzugt in einer Menge von 0,005 bis 10 Gew.-%, vorzugsweise von 0,1 bis 5 Gew.-%, jeweils bezogen auf das gesamte Mittel.

Die erfindungsgemäßen, kosmetischen Mittel enthalten die Kupplerkomponenten bevorzugt in einer Menge von 0,005 bis 10 Gew.-%, vorzugsweise von 0,1 bis 5 Gew.-%, jeweils bezogen auf das gesamte Mittel.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (IVa),

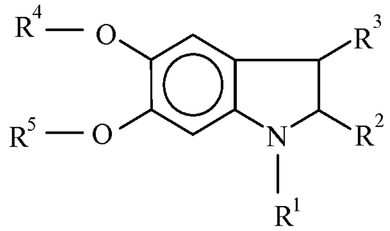

(IVa)

in der unabhängig voneinander

- $R^1$ steht für Wasserstoff, eine $C_1$-$C_4$-Alkylgruppe oder eine $C_1$-$C_4$-Hydroxy-alkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe, und
- $R^5$ steht für eine der unter $R^4$ genannten Gruppen,

sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IVb),

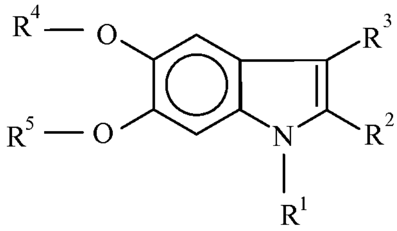

(IVb)

in der unabhängig voneinander

- $R^1$ steht für Wasserstoff, eine $C_1$-$C_4$-Alkylgruppe oder eine $C_1$-$C_4$-Hydroxyalkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe, und
- $R^5$ steht für eine der unter $R^4$ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Bevorzugte direktziehende Farbstoffe, die in den kosmetischen Mitteln als farbverändernde Komponente Verwendung finden, sind Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die kosmetischen Mittel einen kationischen direktziehenden Farbstoff enthalten. Besonders

bevorzugt sind dabei

(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

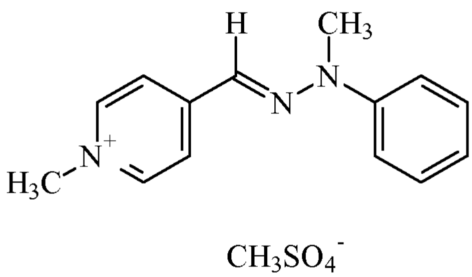
(DZ1)

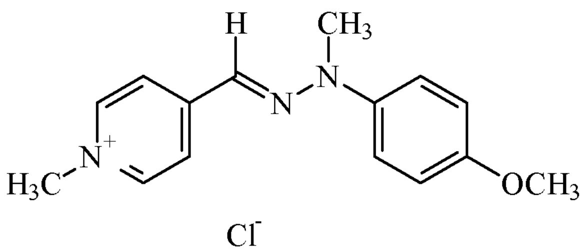
(DZ2)

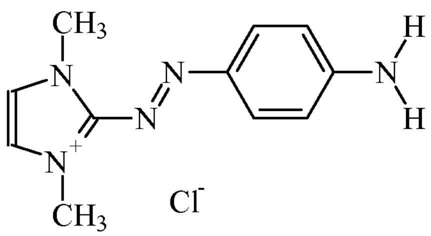
(DZ3)

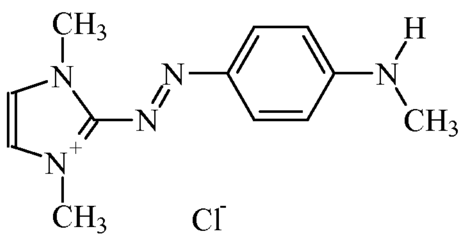
(DZ4)

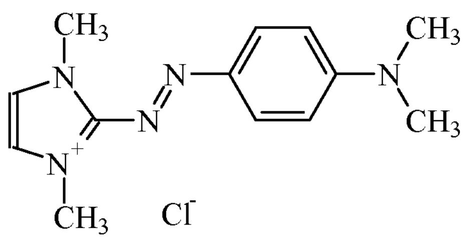
(DZ5)

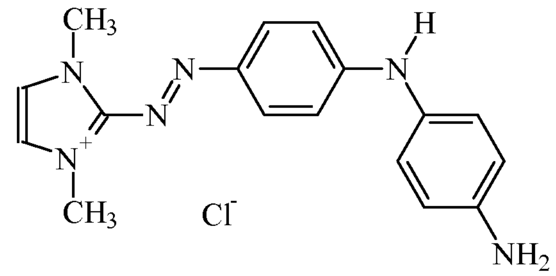

(DZ6)

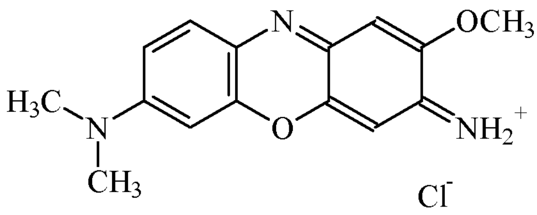

(DZ7)

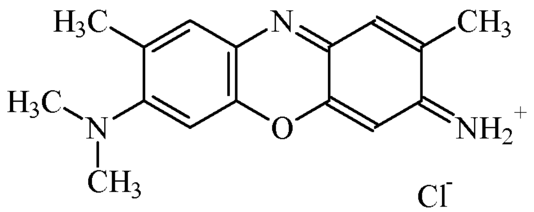

(DZ8)

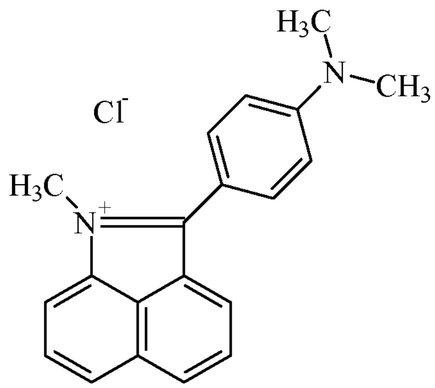

(DZ9)

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Die kosmetischen Mittel enthalten die direktziehenden Farbstoffe bevorzugt in einer Menge von 0,01 bis 20 Gew.-%, bezogen auf das anwendungsbereite Mittel.

Weiterhin können die erfindungsgemäßen kosmetischen Mittel auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Das eigentliche oxidative Färbemittel wird bei getrennter Lagerung der Farbstoffvorprodukte und des Oxidationsmittels unmittelbar vor der Anwendung durch Mischen hergestellt. In einer bevorzugten Ausführungsform wird daher das kosmetische Mittel vor der Applikation aus einer Zusammensetzung, enthaltend in einem kosmetischen Träger mindestens eine farbverändernde Komponente, und einer weiteren Zusammensetzung, enthaltend in einem kosmetischen Träger mindestens ein Oxidationsmittel, gemischt.

Bei einer Anwendung von Oxidationsmitteln wird die anwendungsfertige Zubereitung zweckmäßigerweise unmittelbar vor der Anwendung durch Mischung einer Zusammensetzung, enthaltend das Oxidationsmittel mit der Zusammensetzung, enthaltend die farbverändernden Komponenten, hergestellt. Das dabei entstehende gebrauchsfertige

Haarpräparat sollte bevorzugt einen pH-Wert im Bereich von 6 bis 12, insbesondere von pH 7,5 bis 10, aufweisen.

In einer weiteren bevorzugten Ausführungsform der Erfindung kann die Wirkung durch Zusatz von mindestens einem Fettstoff weiter optimiert werden. Unter Fettstoffen sind zu verstehen Fettsäuren, Fettalkohole, natürliche und synthetische Wachse, welche sowohl in fester Form als auch flüssig in wäßriger Dispersion vorliegen können, und natürliche und synthetische kosmetische Ölkomponenten zu verstehen. Kosmetische Mittel dieser Ausführungsform liegen bevorzugt als Öl-in-Wasser-Emulsion, Wasser-in-Öl-Emulsion oder Dispersion vor.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. In einer bevorzugten Ausführungsform beträgt die Menge 0,5 - 10 Gew.%, wobei ganz besonders vorteilhaft Mengen von 1 - 5 Gew.% sind.

Als Fettalkohole können eingesetzt werden gesättigte, ein- oder mehrfach ungesättigte, verzweigte oder unverzweigte Fettalkohole mit $C_6$ - $C_{30}$-, bevorzugt $C_{10}$ - $C_{22}$- und ganz besonders bevorzugt $C_{12}$ - $C_{22}$- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden. Die Fettalkohole werden in Mengen von 0,1 - 20 Gew.-%, bezogen auf die gesamte Zubereitung, bevorzugt in Mengen von 0,1 - 10 Gew.-% eingesetzt.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Zu den natürlichen und synthetischen kosmetischen Ölkörpern, welche die Wirkung des erfindungsgemäßen Wirkstoffes steigern können, sind beispielsweise zu zählen:

- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyln-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.
- Esteröle. Unter Esterölen sind zu verstehen die Ester von $C_6$ - $C_{30}$ - Fettsäuren mit $C_2$ - $C_{30}$ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).

- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Mono,- Di- und Trifettsäureester von gesättigten und/oder ungesättigten linearen und/oder verzweigten Fettsäuren mit Glycerin, wie beispielsweise Monomuls® 90-018, Monomuls® 90-L12 oder Cutina® MD.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Die Gesamtmenge an Öl- und Fettkomponenten in den erfindungsgemäßen Mitteln beträgt üblicherweise 6 - 45 Gew.-%, bezogen auf das gesamte Mittel. Mengen von 10- 35 Gew.-% sind erfindungsgemäß bevorzugt.

Ferner können die erfindungsgemäßen kosmetischen Mittel weitere Wirk-, Hilfs- und Zusatzstoffe, wie beispielsweise nichtionische Polymere; kationische Polymere; zwitterionische und amphotere Polymere; anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/ Butylmaleat/Isobornylacrylat-Copolymere, Methylvinylether/Malein-säureanhydrid-Copolymere und Acrylsäure/Ethylacrylat/N-tert.Butyl-acrylamid-Terpolymere; haarkonditionierende Verbindungen wie Phospholipide; Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate; Parfümöle, Dimethylisosorbid und Cyclodextrine; faserstrukturverbessernde Wirkstoffe, insbesondere Mono-, Di-und Oligosaccharide wie beispielsweise Glucose, Galactose, Fructose, Fruchtzucker und Lactose; kationische Tenside; Entschäumer; Antischuppenwirkstoffe ; Lichtschutzmittel, insbesondere derivatisierte Benzophenone, Zimtsäure-Derivate und Triazine; Wirkstoffe wie Allantoin, Pyrrolidoncarbonsäuren und deren Salze sowie Bisabolol; Vitamine, Provitamine und Vitaminvorstufen, insbesondere solche der Gruppen A, $B_3$, $B_5$, $B_6$, C, E, F und H; Pflanzenextrakte insbesondere die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng oder Ingwerwurzel; Cholesterin; Konsistenzgeber; Fette und Wachse; Komplexbildner; Quell- und Penetrationsstoffe; Trübungsmittel; Perlglanzmittel; festförmige Pigmente; Stabilisierungsmittel für Wasserstoffperoxid und andere Oxidationsmittel; Treibmittel; Antioxidantien; enthalten.

Ein zweiter Erfindungsgegenstand ist ein Verfahren zur oxidativen Haarbehandlung, in dem ein erfindungsgemäßes kosmetisches Mittel auf das Haar aufgetragen wird und nach einer Einwirkungszeit vom Haar gespült wird.

Die Einwirkungszeit des erfindungsgemäßen Mittels beträgt bevorzugt 1 bis 100 Minuten, besonders bevorzugt 5 bis 50 Minuten.

Es ist wiederum erfindungsgemäß bevorzugt das erfindungsgemäße Verfahren im Rahmen einer oxidativen Haarfärbung, der oxidativen Haarbleiche oder der Fixierung im Rahmen einer Dauerwelle anzuwenden. Dabei werden die besagten mittels Propylenoxid modifizierten Stärken gemeinsam mit dem Oxidationsmittel auf dem Haar angewendet.

Das erfindungsgemäße Verfahren ist ein oxidatives Haarfärbeverfahren, bei welchem die besagte mittels Propylenoxid modifizierte Stärke als Bestandteil eines kosmetischen Mittels enthaltend in einem kosmetischen Träger zusätzlich mindestens ein Oxidationsmittel und mindestens ein Oxidationsfarbstoffvorprodukt in einem Schritt angewendet wird.

Zusätzlich kann optional in einem weiteren Schritt des Verfahrens im Rahmen einer Nachbehandlung ein Mittel,

enthaltend in einem kosmetischen Träger mindestens eine das Haar konditionierende Verbindung in Kombination mit mindestens einer besagten mittels Propylenoxid modifizierten Stärke gebildet wird, auf dem Haar angewendet werden.

Die oxidativen Haarfärbemittel dieser Ausführungsform sind bevorzugt Zweikomponenten Mittel. Die erste Komponente enthält in einem kosmetischen Träger mindestens eine Verbindung aus der Gruppe, die gebildet wird, aus mindestens einer besagten mittels Propylenoxid modifizierten Stärke, und mindestens ein Oxidationsfarbstoffvorprodukt. Die zweite Komponente enthält in einem kosmetischen Träger mindestens ein Oxidationsmittel. Diese Komponenten werden bevorzugt getrennt voneinander in jeweils einem Kompartiment konfektioniert und gemeinsam in einer Verpackungseinheit (Kit) bereitgestellt. Kurz vor der Anwendung werden beide Komponenten miteinander vermischt.

Die zuvor beschriebenen Mehrkomponentenmittel zur Durchführung des erfindungsgemäßen Verfahrens können in einer Verpackungseinheit bereitgestellt werden. Die Verpackungseinheit kann mindestens entweder einen Kontainer umfassen, der das Mittel des ersten Erfindungsgegenstandes enthält, oder kann mindestens zwei Kontainer umfassen. Alle Kontainer können auch Kammern eines Mehrkammerbehältnisses sein.

Für den zweiten Erfindungsgegenstand gilt mutatis mutandis das für den ersten Erfindungsgegenstand Gesagte.

**Beispiele**

1.0 oxidative Haarbehandlung

In eine Färbecreme des Handelsprodukts Igora Royal 6-88 (Schwarzkopf) wurden bezogen auf das Gesamtgewicht der Mischung 1,0 Gew.-% PGE-1 (eine mit 5,5 Gew.-% Propylenoxid modifizierte Tapiokastärke mit einem mittleren Molekulargewicht $M_w$ von 700 bis 900 kDa, einer Viskosität von 55000 mPas (43 Gew.-% wässrige Lösung)) eingearbeitet. Die resultierende modifizierte Färbecreme wurde ummittelbar vor der Applikation auf Haarsträhnen (1 g standardisiertes Haar "European natural hair 6/0" Charge # 06/2010, N93 der Firma Kerling International, Deutschland, an einem Ende zum Haarbündel verklebt) mit einer handelsüblichen 6 Gew.-% Wasserstoffperoxid-haltigen Entwicklerdispersion Oxigenta (Schwarzkopf) im Gewichtsverhältnis 1 zu 1 zu einem erfindungsgemäßen Haarfärbemittel gemischt.

Das anwendungsbereite Färbemittel wurde danach im Gewichtsverhältnis 4 g Färbemittel auf 1 g Haar auf eine Haarsträhne aufgetragen, 30 Minuten bei 32°C einwirken gelassen und von der Faser gespült. Es wurden insgesamt 12 Haarsträhnen damit gefärbt. Darüber hinaus wurden 12 Haarsträhnen nach obiger Vorgehensweise mit dem obigen Handelsprodukt ohne Zusatz der besagten Tapiokastärke (nicht erfindungsgemäß) eingefärbt.

2.0 Messung der Kämmarbeit

Die Haarsträhnen wurden mit Wasser durch besprühen angefeuchtet und 3x manuell vorgekämmt. Vor der Anwendung der oxidativen Haarbehandlungsmittel wurden die Messungen wiederholt. Als Wert für die Kämmarbeit wurde jeweils das arithmetische Mittel gebildet.

Tabelle 1: Nasskämmarbeiten

| | Kämmarbeit, vorher [mJ] | Kämmarbeit, nachher [mJ] |
|---|---|---|
| Handelsprodukt | 267 | 388 |
| erfindungsgemäßes Mittel | 269 | 327 |

Die Daten in Tabelle 1 zeigen, dass der Einsatz der besagten Stärken in oxidativen Haarbehandlungsmitteln die durch die oxidative Haarbehandlung hervorgerufene Steigerung der Kämmarbeit reduziert.

3.0 oxidative Haarbehandlungsmittel

Die folgenden Rezepturen wurden unter Anwendung bekannter Herstellungsverfahren bereitgestellt. Dabei wurden nachstehende Handelsprodukte als Rohstoffe verwendet:

| | |
|---|---|
| Hydrenol® D | $C_{16}$-$C_{18}$-Fettalkohol (INCI-Bezeichnung: Cetearyl alcohol) (Cognis Deutschland) |
| Lorol® techn. | $C_{12}$-$C_{18}$-Fettalkohol (INCI-Bezeichnung: Coconut alcohol) (Cognis Deutschland) |
| Stenol® 16/18 | $C_{16-18}$-Fettalkohol (INCI-Bezeichnung: Cetearyl Alcohol) (Cognis) |
| Lorol® 16 | INCI-Bezeichnung: Cetyl Alcohol (Cognis) |

(fortgesetzt)

| | |
|---|---|
| Eumulgin® B1 | Cetylstearylakohol mit 12 EO-Einheiten (INCI-Bezeichnung: Ceteareth-12) (Cognis Deutschland) |
| Eumulgin® B2 | Cetylstearylalkohol mit ca. 20 EO-Einheiten (INCI-Bezeichnung: Ceteareth-20) (Cognis Deutschland) |
| Edenor® C14 | Myristinsäure (INCI-Bezeichnung: Myristic Acid) (Cognis) |
| Turpinal® SL | 1-Hydroxyethan-1,1-diphosphonsäure (INCI-Bezeichnung: Etidronic Acid, Aqua (Water)) (Solutia) |
| Plantapon® LGC | Alkylpolyglucosid-Carboxylat Natriumsalz; 30 % Aktivsubstanz (Cognis Deutschland) |
| Texapon® NSO UP | Natriumlaurylethersulfat (27 % Aktivsubstanz; INCI: Sodium Laureth Sulfate) (Hersteller: COGNIS) |
| Texapon®K14S70C | Laurylmyristylethersulfat-Natrium-Salz (ca. 68% bis 73% Aktivsubstanzgehalt'; INCI-Bezeichnung: Sodium Myreth Sulfate) (Cognis) |
| Disponil® FES 77 IS | Fettalkoholethersulfat (ca. 31-33% Aktivsubstanzgehalt in Wasser; INCI-Bezeichnung: Sodium Coceth-30 Sulfate) (Cognis) |
| Akypo® Soft 45 NV | 2-($C_{12-14}$-Fettalkoholethoxylat (4.5 EO))-essigsäure Natriumsalz; 21% Aktivsubstanz; INCI-Bezeichnung: Sodium Laureth-5 Carboxylate (KAO) |
| Gluadin® W 40 | Weizenproteinhydrolysat (mind. 40% Festkörper; INCI-Bezeichnung: Aqua (Water), Hydrolyzed Wheat Protein, Sodium Benzoate, Phenoxyethanol, Methylparaben, Propylparaben) (Cognis) |
| Plantacare® 1200 UP | $C_{12-16}$-Fettalkohol-1.4-glucosid (ca. 50-53% Aktivsubstanzgehalt; INCI-Bezeichnung: Lauryl Glucoside, Aqua (Water)) (Cognis) |
| Lamesoft® PO 65 | Alkylpolyglucosid Ölsäuremonoglycerid Gemisch (ca. 65-70% Festkörper; INCI-Bezeichnung: Coco-Glucoside, Glyceryl Oleate, Aqua (Water)) (Cognis) |
| Aculyn® 33 | 30 Gew.-% Aktivsubstanz in Wasser (INCI-Bezeichnung: Acrylates Copolymer) (Rohm & Haas) |
| DOW Corning® DB 110 A | nichtionogene Silikonemulsion (10 Gew.-% Aktivsubstanz) (INCI-Bezeichnung: Dimethicone) (Dow Corning) |
| Aristoflex® AVC | Copolymer von Vinylpyrrolidon und Ammoniumacryloyldimethyltaurat; (INCI-Bezeichnung: Ammonium Acryloyldimethyltaurate/VP Copolymer, t-Butyl Alcohol) (Clariant) |
| Polymer® W 37194 | ca. 20Gew.-% Aktivsubstanzgehalt in Wasser; INCI-Bezeichnung: Acrylamidopropyltrimonium Chloride/Acrylates Copolymer (Stockhausen) |
| Cremophor® A 25 | $C_{16-18}$-Fettalkohol ethoxyliert mit 25 Einheiten Ethylenoxid (INCI-Bezeichnung: Ceteareth-25) (BASF) |

3.2 oxidative Färbemittel

Die Färbemittel F1 bis F16 bestehen jeweils aus einer Färbecreme und einem dazugehörigen Entwickler. Zur Herstellung des anwendungsbereiten Färbemittels wurden 50 g der Färbecreme unter Rühren mit 50 g des entsprechenden Entwicklers gemischt. Bei Applikation auf Kopfhaar und einer Einwirkungszeit von 30 Minuten wurde nach dem Spülen und Trocknen ein hervorragendes Färbeergebnis beobachtet. Die Haare hatten ein gepflegtes äußeres Erscheinungsbild, einen natürlichen Glanz und ließen sich leicht kämmen.

3.2.1 Färbecremes

| | F1 | F2 | F3 | F4 | F5 | F6 | F7 | F8 |
|---|---|---|---|---|---|---|---|---|
| Hydrenol D | 5,50 | 5,50 | 5,50 | 5,50 | 5,50 | 5,50 | 5,50 | 5,50 |
| Lorol 16 | 2,00 | 2,00 | 2,00 | 2,00 | 2,00 | 2,00 | 2,00 | 2,00 |
| Eumulgin B1 | 0,50 | 0,50 | 0,50 | 0,50 | 0,50 | 0,50 | 0,50 | 0,50 |
| Eumulgin B2 | 0,50 | 0,50 | 0,50 | 0,50 | 0,50 | 0,50 | 0,50 | 0,50 |

(fortgesetzt)

| | F1 | F2 | F3 | F4 | F5 | F6 | F7 | F8 |
|---|---|---|---|---|---|---|---|---|
| Lamesoft PO 65 | 2,00 | 2,00 | 2,00 | 2,00 | 2,00 | 2,00 | 2,00 | 2,00 |
| Akypo Soft 45 NV | 10,00 | 10,00 | 10,00 | 10,00 | 10,00 | 10,00 | 10,00 | 10,00 |
| Texapon K 14 S 70% | 2,80 | 2,80 | 2,80 | 2,80 | 2,80 | 2,80 | 2,80 | 2,80 |
| Ammoniak 25% | 6,50 | 6,50 | 6,50 | 6,50 | 6,50 | 6,50 | 6,50 | 6,50 |
| Turpinal SL | 0,20 | 0,20 | 0,20 | 0,20 | 0,20 | 0,20 | 0,20 | 0,20 |
| p-Toluylendiamisulfat | 2,50 | 0,40 | 0,30 | 0,05 | 0,30 | 2,50 | 0,40 | 0,30 |
| Resorcin | 0,40 | 0,10 | 1,00 | 0,02 | 0,03 | 0,40 | 0,10 | 1,00 |
| 2,4-Diaminophenoxyethanol 2HCl | 2,00 | - | - | - | - | 2,00 | - | - |
| 2-Methylresorcin | - | 1,00 | 0,03 | - | 0,50 | - | 1,00 | 0,03 |
| 2,4,5,6-Tetraaminopyrimidinsulfat | - | 1,50 | - | - | - | - | 1,50 | - |
| Bis-(5-Amino-2-hydroxyphenyl)methan 2HCl | - | 0,06 | - | - | - | - | 0,06 | - |
| m-Aminophenol | - | - | 0,01 | 0,03 | 0,02 | - | - | 0,01 |
| 4-Chlorresorcin | - | - | 0,60 | 0,01 | 0,10 | - | - | 0,60 |
| Ascorbinsäure | 0,10 | 0,10 | 0,10 | 0,10 | 0,10 | 0,10 | 0,10 | 0,10 |
| Natriumsulfit 96% | 0,40 | 0,40 | 0,40 | 0,40 | 0,40 | 0,40 | 0,40 | 0,40 |
| PGE-1 | 1,00 | 1,50 | 2,00 | 0,50 | 0,75 | 1,00 | 1,00 | 2,50 |
| Aqua | <----------------------------ad. 100-------------------------------> | | | | | | | |
| | F9 | F10 | F11 | F12 | F13 | F14 | F15 | F16 |
| Cetearyl Alkohol | 8,00 | 8,00 | 8,00 | 8,00 | 8,00 | 8,00 | 8,00 | 8,00 |
| Lorol | 3,00 | 3,00 | 3,00 | 3,00 | 3,00 | 3,00 | 3,00 | 3,00 |
| Eumulgin B2 | 2,00 | 2,00 | 2,00 | 2,00 | 2,00 | 2,00 | 2,00 | 2,00 |
| Ascorbinsäure | 0,50 | 0,50 | 0,50 | 0,50 | 0,50 | 0,50 | 0,50 | 0,50 |
| Natriumsulfit 96% | 0,50 | 0,50 | 0,50 | 0,50 | 0,50 | 0,50 | 0,50 | 0,50 |
| Turpinal SL | 0,20 | 0,20 | 0,20 | 0,20 | 0,20 | 0,20 | 0,20 | 0,20 |
| Gluadin W 40 | 4,00 | 4,00 | 4,00 | 4,00 | 4,00 | 4,00 | 4,00 | 4,00 |
| Ammoniak 25% | 6,00 | 6,00 | 6,00 | 6,00 | 6,00 | 6,00 | 6,00 | 6,00 |
| Resorcin | 0,10 | 8,00 | 8,00 | 8,00 | 8,00 | 8,00 | 8,00 | 8,00 |
| p-Toluylendiaminsulfat | 0,15 | 0,70 | 1,80 | 0,15 | 0,70 | 1,80 | 0,70 | 1,80 |
| 4-Chlorresorcin | 0,03 | 0,02 | 0,50 | 0,03 | 0,02 | 0,50 | 0,02 | 0,50 |
| 2-Amino-2-metylamino-6-methoxypyridin | 0,01 | - | - | 0,01 | - | - | - | - |
| 2,6-Dihydroxy-3,4-Dimethylpyridin | - | 0,02 | - | - | 0,02 | - | 0,02 | - |
| 2,7-Dihydroxynaphthalin | - | 0,01 | - | - | 0,01 | - | 0,01 | - |
| 2-Methylresorcin | - | 0,05 | - | - | 0,05 | - | 0,05 | - |
| m-Aminophenol | - | 0,10 | - | - | 0,10 | - | 0,10 | - |
| 2,4-Diaminophenoxyethanol 2 HCl | - | 0,01 | - | - | 0,01 | - | 0,01 | - |
| 3-Amino-2-methylamino-6-methoxypyridin | - | 0,01 | 0,10 | - | 0,01 | 0,10 | 0,01 | 0,10 |
| 1,3-Bis-(2,4-diaminophenoxy)propan 4HCl | - | - | 0,30 | - | - | 0,30 | - | 0,30 |
| PGE-1 | 1,00 | 1,50 | 2,00 | 1,00 | 0,50 | 0,25 | 1,00 | 1,25 |

(fortgesetzt)

| | F1 | F2 | F3 | F4 | F5 | F6 | F7 | F8 |
|---|---|---|---|---|---|---|---|---|
| Wasser | <---------------------------------ad. 100---------------------------------> | | | | | | | |

3.2.2 Entwickler

| | F1 | F2 | F3 | F4 | F5 | F6 | F7 | F8 |
|---|---|---|---|---|---|---|---|---|
| Dipicolinsäure | 0,10 | 0,10 | 0,10 | 0,10 | 0,10 | 0,10 | 0,10 | 0,10 |
| Dinatriumpyrophosphat | 0,03 | 0,03 | 0,03 | 0,03 | 0,03 | 0,03 | 0,03 | 0,03 |
| Turpinal SL | 1,50 | 1,50 | 1,50 | 1,50 | 1,50 | 1,50 | 1,50 | 1,50 |
| Texapon NSO UP | 2,00 | 2,00 | 2,00 | 2,00 | 2,00 | 2,00 | 2,00 | 2,00 |
| Aculyn 33A | 12,00 | 12,00 | 12,00 | 12,00 | 12,00 | 12,00 | 12,00 | 12,00 |
| Wasserstoffperoxid 50% | 12,00 | 6,00 | 12,00 | 6,00 | 12,00 | 6,00 | 12,00 | 6,00 |
| Ammoniak | 0,62 | 0,62 | 0,62 | 0,62 | 0,62 | 0,62 | 0,62 | 0,62 |
| Wasser | <-------------------------------ad. 100-------------------------------> | | | | | | | |

| | F9 | F10 | F11 | F12 | F13 | F14 | F15 | F16 |
|---|---|---|---|---|---|---|---|---|
| Lorol C16 | 3,50 | 3,50 | 3,50 | 3,50 | 3,50 | 3,50 | 3,50 | 3,50 |
| Eumulgin B2 | 1,00 | 1,00 | 1,00 | 1,00 | 1,00 | 1,00 | 1,00 | 1,00 |
| Disponil FES 77 IS | 2,50 | 2,50 | 2,50 | 2,50 | 2,50 | 2,50 | 2,50 | 2,50 |
| Wasserstoffperoxid 50% | 24,00 | 20,00 | 12,00 | 24,00 | 20,00 | 12,00 | 24,00 | 20,00 |
| Turpinal SL | 1,50 | 1,50 | 1,50 | 1,50 | 1,50 | 1,50 | 1,50 | 1,50 |
| Aculyn 33A | 10,0 | 10,0 | 10,0 | 10,0 | 10,0 | 10,0 | 10,0 | 10,0 |
| Wasser | <------------------------------------ad. 100------------------------------------> | | | | | | | |

3.3 ammoniakfreie oxidative Färbemittel

Die Färbemittel F17 bis F25 bestehen jeweils aus einer Färbecreme und dem nachfolgend genannten Entwickler. Zur Herstellung des anwendungsbereiten Färbemittels wurden 50 g der Färbecreme unter Rühren mit 50 g des Entwicklers gemischt. Bei Applikation auf Kopfhaar und einer Einwirkungszeit von 30 Minuten wurde nach dem Spülen und Trocknen ein hervorragendes Färbeergebnis beobachtet. Die Haare hatten ein gepflegtes Äußeres, einen natürlichen Glanz und ließen sich leicht kämmen.

3.3.1 Farbecremes

| | F17 | F18 | F19 | F20 | F21 | F22 | F23 | F24 | F25 |
|---|---|---|---|---|---|---|---|---|---|
| Hydrenol D | 7,15 | 7,15 | 7,15 | 7,15 | 7,15 | 7,15 | 7,15 | 7,15 | 7,15 |
| Lorol | 2,60 | 2,60 | 2,60 | 2,60 | 2,60 | 2,60 | 2,60 | 2,60 | 2,60 |
| Eumulgin B1 | 0,65 | 0,65 | 0,65 | 0,65 | 0,65 | 0,65 | 0,65 | 0,65 | 0,65 |
| Eumulgin B2 | 0,65 | 0,65 | 0,65 | 0,65 | 0,65 | 0,65 | 0,65 | 0,65 | 0,65 |

(fortgesetzt)

| | F17 | F18 | F19 | F20 | F21 | F22 | F23 | F24 | F25 |
|---|---|---|---|---|---|---|---|---|---|
| Akypo Soft 45 NV | 10,0 | 10,0 | 10,0 | 10,0 | 10,0 | 10,0 | 10,0 | 10,0 | 10,0 |
| Texapon K 14 S 70% | 2,80 | 2,80 | 2,80 | 2,80 | 2,80 | 2,80 | 2,80 | 2,80 | 2,80 |
| Plantacare 1200 | 2,00 | 2,00 | 2,00 | 2,00 | 2,00 | 2,00 | 2,00 | 2,00 | 2,00 |
| Produkt W 37194 | 3,75 | 3,75 | 3,75 | 3,75 | 3,75 | 3,75 | 3,75 | 3,75 | 3,75 |
| 2-Aminoethan-1-ol | 3,10 | 2,80 | 2,00 | 3,10 | 2,80 | 2,00 | 3,10 | 2,80 | 2,00 |
| L-Arginin | - | 1,00 | - | - | 1,00 | - | - | 1,00 | - |
| 2-Amino-2-metylpropanol | - | - | 1,50 | - | - | 1,50 | - | - | 1,50 |
| Natriumchlorid | 0,50 | 0,50 | 0,50 | 0,50 | 0,50 | 0,50 | 0,50 | 0,50 | 0,50 |
| Natriumsulfit | 0,40 | 0,40 | 0,40 | 0,40 | 0,40 | 0,40 | 0,40 | 0,40 | 0,40 |
| Ascorbinsäure | 0,10 | 0,10 | 0,10 | 0,10 | 0,10 | 0,10 | 0,10 | 0,10 | 0,10 |
| Turpinal SL | 0,20 | 0,20 | 0,20 | 0,20 | 0,20 | 0,20 | 0,20 | 0,20 | 0,20 |
| Natronwasserglas 40/42 | 0,50 | 0,50 | 0,50 | 0,50 | 0,50 | 0,50 | 0,50 | 0,50 | 0,50 |
| Kaliumhydroxid 50% | 1,00 | 1,00 | 1,00 | 1,00 | 1,00 | 1,00 | 1,00 | 1,00 | 1,00 |
| Glycin | 1,00 | 1,00 | 1,00 | 1,00 | 1,00 | 1,00 | 1,00 | 1,00 | 1,00 |
| PGE 1 | 1,00 | 1,00 | 1,00 | 3,00 | 0,50 | 4,50 | 0,45 | 2,00 | 1,00 |
| p-Toluylendiamisulfat | 0,40 | 0,40 | 0,40 | 0,40 | 0,40 | 0,40 | 0,40 | 0,40 | 0,40 |
| 2,7-Dihydroxynaphtalin | 0,03 | 0,03 | 0,03 | 0,03 | 0,03 | 0,03 | 0,03 | 0,03 | 0,03 |
| Resorcin | 0,12 | 0,12 | 0,12 | 0,12 | 0,12 | 0,12 | 0,12 | 0,12 | 0,12 |
| 4-Chlorresorcin | 0,06 | 0,06 | 0,06 | 0,06 | 0,06 | 0,06 | 0,06 | 0,06 | 0,06 |
| 2-Methylresorcin | 0,02 | 0,02 | 0,02 | 0,02 | 0,02 | 0,02 | 0,02 | 0,02 | 0,02 |
| m-Aminophenol | 0,02 | 0,02 | 0,02 | 0,02 | 0,02 | 0,02 | 0,02 | 0,02 | 0,02 |
| Wasser | <--------------------------------ad 100--------------------------------> | | | | | | | | |

3.3.2 Entwickler

| | |
|---|---|
| Dipicolinsäure | 0,10 |
| Dinatriumpyrophosphat | 0,03 |
| Turpinal SL | 1,50 |
| Texapon NSO UP | 2,00 |
| Aculyn 33A | 12,00 |
| Wasserstoffperoxid 50% | 12,00 |
| Wasser | ad. 100 |

**Patentansprüche**

**1.** Mittel, enthaltend in einem kosmetischen Träger eine Kombination aus

(i) mindestens einem Oxidationsmittel, wobei das Oxidationsmittel Wasserstoffperoxyd und/oder mindestens ein Anlagerungsprodukt von Wasserstoffperoxyd an anorganische oder organische Verbindungen ist und

(ii) mindestens einer mittels Propylenoxid modifizierten Stärke, welche ein mittleres Molekulargewicht (Gewichtsmittel) von 50 bis 2500 kDa und einen Propylenoxidgehalt von 0,1 bis 20,0 Gew.-% (bezogen auf das Gewicht der mittels Propylenoxid modifizierten Stärke) besitzt, wobei die mittels Propylenoxid modifizierte Stärke unvernetzt ist,

wobei es als farbverändernde Komponente mindestens ein Oxidationsfarbstoffvorprodukt vom Typ der Entwicklerkomponenten und gegebenenfalls zusätzlich mindestens eine Kupplerkomponente enthält.

2. Mittel nach Anspruch 1, **dadurch gekennzeichnet, dass** besagte mittels Propylenoxid modifizierten Stärke ein mittleres Molekulargewicht (Gewichtsmittel) von 100 bis 2000 kDa, insbesondere von 500 bis 1800 kDa, ganz besonders bevorzugt von 700 bis 1000 kDa, aufweisen.

3. Mittel nach einem der Ansprüche 1 oder 2, **dadurch gekennzeichnet, dass** besagte mittels Propylenoxid modifizierte Stärke - bezogen auf das Gewicht der modifizierten Stärke - einen Propylenoxidgehalt von 2 bis 12 Gew.-%, besonders bevorzugt einen Propylenoxidgehalt von 3,0 bis 10,0 Gew.-%, ganz besonders bevorzugt einen Propylenoxidgehalt von 4,0 bis 6,0 Gew.-%, aufweist.

4. Mittel nach einem der Ansprüche 1 bis 3, **dadurch gekennzeichnet, dass** die besagte mittels Propylenoxid modifizierte Stärke in einer 43 Gew.-%-igen Lösung in Wasser (i.e. einer 43 Gew.-%-igen wässrigen Lösung) eine Viskosität im Bereich von 150 bis 1500000 mPa.s (Brookfield Viskosimeter, Spindel #7 bei 20°C und 20 U/min), bevorzugt von 3000 bis 200000 mPa.s, besonders bevorzugt von 10000 bis 100000 mPa.s, ganz besonders bevorzugt von 40000 bis 70000 mPa.s, (jeweils gemessen unter den zuvor genannten Bedingungen) aufweist.

5. Mittel nach einem der Ansprüche 1 bis 4, **dadurch gekennzeichnet, dass** es die besagten mittels Propylenoxid modifizierten Stärken bevorzugt in einer Menge von in einer Menge von 0,01 Gew.-% bis 20 Gew.-%, besonders bevorzugt von 0,01 Gew.-% bis 10,0 Gew.-%, ganz besonders bevorzugt von 1 Gew.-% bis 5 Gew.-%, am bevorzugtesten von 0,1 bis 2,0 Gew.-%, jeweils bezogen auf das Gewicht des anwendungsbereiten Mittels, enthält.

6. Verfahren zur oxidativen Haarbehandlung, in dem ein kosmetisches Mittel nach einem der Ansprüche 1 bis 5 auf das Haar aufgetragen wird und nach einer Einwirkungszeit vom Haar gespült wird.

## Claims

1. An agent containing, in a cosmetic carrier, a combination of

(i) at least one oxidizing agent, wherein the oxidizing agent is hydrogen peroxide and/or at least one addition product of hydrogen peroxide to inorganic or organic compounds, and
(ii) at least one propylene-oxide-modified starch which has an average molecular weight (weight average) of from 50 to 2500 kDa and a propylene oxide content of from 0.1 to 20.0 wt.% (based on the weight of the propylene-oxide-modified starch), wherein the propylene-oxide-modified starch is uncrosslinked,

wherein said agent contains at least one oxidation dye precursor of the type of the developer components, as the color-changing component, and optionally additionally at least one coupler component.

2. The agent according to claim 1, **characterized in that** said propylene-oxide-modified starch has an average molecular weight (weight average) of from 100 to 2000 kDa, in particular from 500 to 1800 kDa, very particularly preferably from 700 to 1000 kDa.

3. The agent according to one of claims 1 or 2, **characterized in that** said propylene-oxide-modified starch has, based on the weight of the modified starch, a propylene oxide content of from 2 to 12 wt.%, particularly preferably a propylene oxide content of from 3.0 to 10.0 wt.%, very particularly preferably a propylene oxide content of from 4.0 to 6.0 wt.%.

4. The agent according to one of claims 1 to 3, **characterized in that** said propylene-oxide-modified starch has, in a 43 wt.% solution in water (i.e. in a 43 wt.% aqueous solution), a viscosity in the range of from 150 to 1,500,000 mPa.s (Brookfield viscometer, spindle #7 at 20°C and 20 RPM), preferably from 3,000 to 200,000 mPa.s, particularly preferably from 10,000 to 100,000 mPa.s, very particularly preferably from 40,000 to 70,000 mPa.s (in each case

measured under the above-mentioned conditions).

**5.** The agent according to one of claims 1 to 4, **characterized in that** said propylene-oxide-modified starch is contained preferably in a quantity of from 0.01 wt.% to 20 wt.%, particularly preferably from 0.01 wt.% to 10.0 wt.%, very particularly preferably from 1 wt.% to 5 wt.%, most preferably from 0.1 to 2.0 wt.%, in each case based on the weight of the ready-to-apply agent.

**6.** A method for oxidative hair treatment, in which a cosmetic agent according to one of claims 1 to 5 is applied to the hair and rinsed off the hair after a contact time.

## Revendications

**1.** Agent contenant dans un support cosmétique une combinaison

(i) d'au moins un agent oxydant, l'agent oxydant étant le peroxyde d'hydrogène et/ou au moins un produit d'addition de peroxyde d'hydrogène à des composés minéraux ou organiques, et
(ii) au moins un amidon, modifié par de l'oxyde de propylène, ayant un poids moléculaire moyen (moyenne en poids) de 50 à 2500 kDa et une teneur en oxyde de propylène de 0,1 à 20,0% en poids (sur la base du poids de l'amidon modifié par de l'oxyde de propylène), l'amidon modifié par de l'oxyde de propylène étant non réticulé,

l'agent contenant comme composant modificateur de couleur au moins un précurseur de colorant d'oxydation du type composant développeur et éventuellement en outre au moins un composant copulateur.

**2.** Agent selon la revendication 1, **caractérisée en ce que** ledit amidon modifié par de l'oxyde de propylène a un poids moléculaire moyen (moyenne en poids) de 100 à 2000 kDa, en particulier de 500 à 1800 kDa, de préférence de 700 à 1000 kDa.

**3.** Agent selon l'une des revendications 1 ou 2, **caractérisé en ce que** ledit amidon modifié par de l'oxyde de propylène a, sur la base du poids de l'amidon modifié, une teneur en oxyde de propylène allant de 2 à 12 % en poids, de façon particulièrement préférée une teneur en oxyde de propylène allant de 3,0 à 10,0 % en poids, de façon tout particulièrement préférée une teneur en oxyde de propylène allant de 4,0 à 6,0 % en poids.

**4.** Agent selon l'une des revendications 1 à 3, **caractérisée en ce que** ledit amidon modifié par l'oxyde de propylène dans une dilution de 43% en poids dans de l'eau (c'est-à-dire une solution aqueuse de 43% en poids) a une viscosité dans la gamme allant de 150 à 1500000 mPa.s (viscosimètre Brookfield, broche n°7 à 20°C et 20 tr/min), de préférence de 3000 à 200000 mPa.s, de façon particulièrement préférée de 10000 à 100000 mPa.s, de façon tout particulièrement préférée de 40000 à 70000 mPa.s (mesurée à chaque fois dans les conditions susmentionnées).

**5.** Agent selon l'une des revendications 1 à 4, **caractérisée en ce qu'**il contient lesdits amidons modifiés par de l'oxyde de propylène de préférence dans une quantité de 0,01% en poids à 20% en poids, de façon particulièrement préférée de 0,01% en poids à 10,0% en poids, de façon tout particulièrement préférée de 1% en poids à 5% en poids, le plus préférablement de 0,1 à 2,0% en poids, à chaque fois sur la base du poids de l'agent prêt à l'emploi.

**6.** Procédé de traitement capillaire par oxydation dans lequel un agent cosmétique selon l'une des revendications 1 à 5 est appliqué sur les cheveux et rincé des cheveux après un temps d'action.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1568351 A1
- WO 9801109 A1
- WO 2012084904 A1
- US 20050193501 A1
- US 20060075580 A1
- GB 1026978 A
- GB 1153196 A
- DE 2359399
- JP 02019576 A
- WO 9615765 A
- DE 3843892
- DE 4133957
- WO 9408969 A
- WO 9408970 A
- EP 740931 A
- DE 19543988
- EP 998908 A2
- DE 19756454 A